# EUROPÄISCHE PATENTSCHRIFT

(11) **EP 3 402 547 B1**
(45) Veröffentlichungstag und Bekanntmachung des Hinweises auf die Patenterteilung: **06.11.2019**
(21) Anmeldenummer: 17700095.7
(22) Anmeldetag: 05.01.2017
(51) Int. Cl.: A61M 1/34

(54) **VORRICHTUNG ZUR DURCHFÜHRUNG EINER EXTRAKORPORALEN BLUTBEHANDLUNG**
DEVICE FOR CARRYING OUT AN EXTRACORPOREAL BLOOD TREATMENT
DISPOSITIF POUR EFFECTUER UN TRAITEMENT EXTRACORPOREL DU SANG

(30) Priorität: 15.01.2016 DE 102016000367
(43) Veröffentlichungstag der Anmeldung: 21.11.2018
(73) Patentinhaber: Fresenius Medical Care Deutschland GmbH, 61352 Bad Homburg (DE)
(72) Erfinder: BREUEL, Lars, 99310 Witzleben/ OT Achelstädt (DE); KLEWINGHAUS, Jürgen, 61440 Oberursel (DE)
(74) Vertreter: Herrmann, Uwe
(86) Internationale Anmeldenummer: PCT/EP2017/000008
(87) Internationale Veröffentlichungsnummer: WO 2017/121632

(56) Entgegenhaltungen:
- US-A1- 2013 233 394

## Beschreibung

Die Erfindung betrifft eine Vorrichtung zur Durchführung einer extrakorporalen Blutbehandlung, in der dem Patienten eine Substitutionsflüssigkeit verabreicht wird.

In der Plasmapherese wird anhand eines in einem extrakorporalen Blutkreislauf angeordneten Filters Plasma aus dem Blut eines Patienten abgetrennt. Das Verfahren wird beispielsweise zur Behandlung mancher Autoimmunerkrankungen verwendet. Bei einer Variante der Methode, die als Plasmaaustausch bezeichnet wird, wird das entfernte Plasma verworfen und eine Substitutionsflüssigkeit in den extrakorporalen Blutkreislauf gegeben. Bei der Substitutionsflüssigkeit kann es sich beispielsweise um Spenderplasma handeln.

Die Erfindung ist aber nicht auf die Plasmapherese beschränkt sondern allgemein auf Geräte zur extrakorporalen Blutbehandlung anwendbar, in denen dem Patienten eine nicht körpereigene Substitutionsflüssigkeit zugeführt wird.

Die Substitutionsflüssigkeit wird typischerweise in mehreren getrennten Behältnissen wie beispielsweise Beuteln bereitgestellt, die gesondert an eine Substitutionsleitung angeschlossen sind. Dabei ist es üblich, die Fluidverbindung zwischen einem Behältnis und der Substitutionsleitung erst dann zu öffnen, wenn das vorhergehende Behältnis leer ist. So können etwaige Unverträglichkeitsreaktionen des Patienten dem Inhalt eines bestimmten Behältnisses (beispielsweise dem Plasma eines bestimmten Spenders) zugeordnet werden.

Aus der Druckschrift US 2013/2333394 A1 ist beispielsweise eine Vorrichtung zur Durchführung einer extrakorporalen Blutbehandlung bekannt, in der dem Patienten eine Substitutionsflüssigkeit verabreicht wird. Diese bekannte Vorrichtung ist jedoch in ihrem Aufbau recht kompliziert.

Nachteilig ist bei bekannten Vorrichtungen hierbei, dass die Fluidverbindung für jedes einzelne Behältnis manuell geöffnet werden muss, was umständlich, zeitintensiv und personalintensiv ist. Es bedeutet außerdem einen Nutzereingriff zu einem nicht exakt zu bestimmenden Zeitpunkt.

Aufgabe der Erfindung ist es, eine gattungsgemäße Vorrichtung bereitzustellen, in der diese Nachteile vermieden werden.

Vor diesem Hintergrund betrifft die Erfindung eine Vorrichtung zur Durchführung einer extrakorporalen Blutbehandlung, in der dem Patienten eine Substitutionsflüssigkeit verabreicht wird, wobei die Vorrichtung einen extrakorporalen Blutkreislauf und eine in den extrakorporalen, Blutkreislauf mündende Substitutionsleitung umfasst, wobei die Substitutionsleitung eine Pumpe zur Förderung von Substitutionsflüssigkeit in den extrakorporalen Blutkreislauf aufweist und wobei wenigstens zwei und vorzugsweise mehr als zwei Behältnisse zur Bereitstellung einer Substitutionsflüssigkeit separat und saugseitig der Pumpe an die Substitutionsleitung angeschlossen sind. Erfindungsgemäß ist vorgesehen, dass zwischen der Pumpe und wenigstens einem der Behältnisse wenigstens ein Druckventil angeordnet ist, das bei Überschreitung eines Öffnungsdrucks einen Fluss von Substitutionsflüssigkeit zur Pumpe erlaubt.

Vorzugsweise dient die Vorrichtung der Durchführung einer Plasmapheresebehandlung. Bei der Substitutionsflüssigkeit kann es sich beispielsweise um Spenderplasma handeln.

In einer Ausführungsform ist die Anordnung der Druckventile und Behältnisse derart, dass eine Staffelung der Öffnungsdrücke erreicht wird, welche von der Pumpe ausgeübt werden müssen, um Substitutionsflüssigkeit aus unterschiedlichen Behältnissen zu beziehen. Da die Behältnisse saugseitig der Pumpe angeordnet sind, wird von der Pumpe stromabwärts der Ventile ein Unterdruck erzeugt, und der Öffnungsdruck ergibt sich aus der Druckdifferenz zwischen dem Normaldruck stromaufwärts und dem Unterdruck stromabwärts der Ventile.

Die Pumpe bezieht Substitutionsflüssigkeit zunächst aus dem Behältnis, zwischen dem und der Pumpe entweder kein Ventil, das Ventil mit dem niedrigsten Öffnungsdruck oder die geringste Zahl an identischen Ventilen angeordnet ist. Wenn dieses Behältnis geleert wurde, bezieht die Pumpe sodann Substitutionsflüssigkeit aus dem Behältnis, zwischen dem und der Pumpe das Ventil mit dem zweitniedrigsten Öffnungsdruck oder die zweitgeringste Zahl an identischen Ventilen angeordnet ist. Dies setzt sich fort, bis alle für die Behandlung benötigten Behältnisse sequentiell entleert wurden.

In einer Ausführungsform sind die Druckventile stromaufwärts der Anschlüsse der jeweiligen Behältnisse an die Substitutionsleitung angeordnet.

In einer Ausführungsform ist jedem Behältnis wenigstens ein gesondertes Druckventil zugeordnet. Alternativ kann beispielsweise einem der Behältnisse kein Druckventil zugeordnet sein, d.h. allen außer einem der Behältnisse ein Druckventil zugeordnet sein.

In einer Ausführungsform sind unterschiedlichen Behältnissen unterschiedlich viele in Serie geschaltete Druckventile zugeordnet. Diese in Serie geschalteten Druckventile können in einer Ausgestaltung alle denselben Öffnungsdruck aufweisen. Der Öffnungsdruck der in Serie geschalteten Ventile summiert sich auf. So wird trotz der Verwendung identischer Ventile eine Staffelung der Öffnungsdrücke erreicht.

In einer alternativen Ausführungsform sind unterschiedlichen Behältnissen Druckventile mit unterschiedlichem Öffnungsdruck zugeordnet. Auch so kann eine Staffelung der Öffnungsdrücke erreicht werden.

Auch Mischformen sind denkbar, beispielsweise der Einsatz zweier oder mehrerer unterschiedlicher Druckventile mit unterschiedlichen Öffnungsdrücken und die serielle Anordnung dieser Druckventile zum Erreichen eines Öffnungsdrucks, der höher als der Öffnungsdruck desjenigen Einzelventils mit dem höchsten Öffnungsdruck ist.

In einer Ausführungsform sind einige oder alle der Behältnisse parallel an die Substitutionsleitung angeschlossen.

In einer alternativen Ausführungsform sind einige oder alle der Behältnisse seriell an die Substitutionsleitung angeschlossen. Bei einem seriellen Anschluss kann vorgesehen sein, dass die benötigten Öffnungsdrücke mit zunehmender Entfernung der Behältnisse von der Pumpe steigen.

Auch Mischformen sind denkbar, beispielsweise zwei oder mehrere parallel, angeschlossene Stränge, an denen die Behältnisse seriell angeschlossen sind.

Bei einem seriellen Anschluss der Behältnisse an die Substitutionsleitung sind in einer Ausführungsform die Druckventile zwischen den Anschlüssen der Behältnisse an die Substitutionsleitung angeordnet. Vorzugsweise sind Druckventile zwischen allen Anschlüssen angeordnet. In dieser Ausführungsform addieren sich die benötigten Öffnungsdrücke für den Zugriff auf diejenigen Behältnisse, zwischen denen und der Pumpe mehrere Druckventile in der Substitutionsleitung liegen. Diese Ausführungsform hat den Vorteil, dass die Vorrichtung gegebenenfalls mit weniger Druckventilen auskommen kann.

In einer Ausführungsform ist zwischen der Pumpe und dem ersten Behältnis der Serie kein Druckventil angeordnet. Unter dem ersten Behältnis wird das Behältnis verstanden, dessen Anschluss an die Substitutionsleitung näher an der Pumpe liegt als die Anschlüsse aller anderen Behältnisse. Alternativ kann auch zwischen dem ersten Behältnis der Serie und der Pumpe ein Druckventil angeordnet sein.

In einer Ausführungsform weisen alle Druckventile denselben Öffnungsdruck auf. Dies kann in allen vorgenannten Ausführungsformen der Fall sein. Dadurch ergibt sich hierdurch eine Kostenersparnis durch die Verwendung identischer Teile.

In einer Ausführungsform handelt es sich bei den Druckventilen um Rückschlagventile, die einen Fluss von Substitutionsflüssigkeit nur in Richtung der Pumpe erlauben.

In einer Ausführungsform sind die Behältnisse kompressibel. Vorzugsweise handelt es sich um Kunststoffbeutel. In diesem Fall kann sich das Beutelvolumen mit dem sinkenden enthaltenen Flüssigkeitsvolumen beim Leersaugen durch die Pumpe verkleinern.

Was die Größe des Öffnungsdrucks betrifft, so kann beispielsweise vorgesehen sein, dass der Öffnungsdruck einzelner Ventile zwischen 20 und 300 mbar und vorzugsweise bei zwischen 50 und 100 mbar liegt.

Im Rahmen der vorliegenden Offenbarung ist ferner angedacht, ein Verfahren, das nicht Teil der Erfindung ist, zur extrakorporalen Blutbehandlung unter Verwendung einer erfindungsgemäßen Vorrichtung bereitzustellen, wobei Substitutionsflüssigkeit in den extrakorporalen Blutkreislauf bzw. in eine mit diesem in Fluidverbindung stehende Substitutionsleitung verabreicht wird und wobei die Substitutionsflüssigkeit derart sequentiell aus den unterschiedlichen Behältnissen der Vorrichtung gefördert wird, dass die Förderung aus einem Behältnis erst dann einsetzt, wenn das vorhergehende Behältnis leer ist.

Weitere Einzelheiten und Vorteile der Erfindung ergeben sich aus den nachfolgend anhand der Figuren erklärten Ausführungsbeispielen. In den Figuren zeigen:
- Figur 1:: ein Flussbild einer Vorrichtung für den Plasmaaustausch;
- Figur 2:: eine Ausführungsform der erfindungsgemäßen Vorrichtung mit paralleler Anordnung der Behältnisse; und
- Figur 3:: eine Ausführungsform der erfindungsgemäßen Vorrichtung mit serieller Anordnung der Behältnisse.

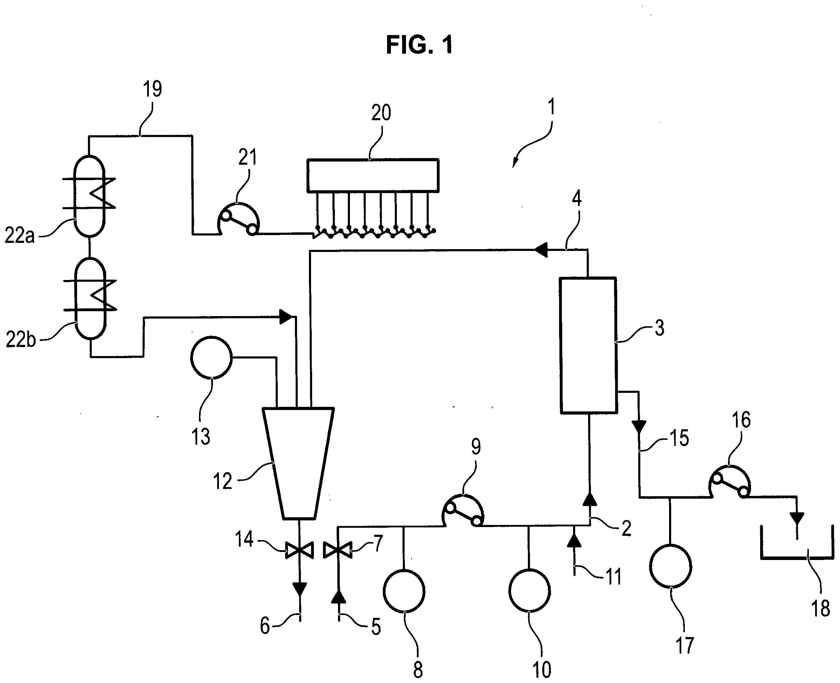

Der in Figur 1 schematisch dargestellte extrakorporale Blutkreislauf einer Ausführungsform einer erfindungsgemäßen Vorrichtung ist mit dem Bezugszeichen 1 gekennzeichnet.

Er umfasst eine arterielle Leitung 2, einen Plasmafilter 3 und eine venöse Leitung 4. An der Vorlaufseite der arteriellen Leitung 2 ist ein arterieller Port 5 und an der Rücklaufseite der venösen Leitung 4 ein venöser Port 6 angeordnet. Diese Ports 5 und 6, bei denen es sich beispielsweise um Nadeln handeln kann, dienen zum Anschluss der Vorrichtung an einen Patienten.

In der arteriellen Leitung 2 sind in Flussrichtung gestaffelt eine arterielle Klemme 7, ein Drucksensor 8 zur Messung des arteriellen Drucks, eine Blutpumpe 9, ein weiterer Drucksensor 10 zur Messung des Pumpdrucks und eine Leitung 11 zur Zufuhr eines Antikoagulationsmittels wie beispielsweise Heparin angeordnet.

In der venösen Leitung 4 befinden sich eine Tropfkammer 12, ein Drucksensor 13 zur Messung des venösen Drucks und eine venöse Klemme 14.

Der Plasmafilter 3 umfasst eine semipermeable Membran, die den extrakorporalen Blutkreislauf 1 von einem Abflusssystem 15 für aus dem Blut abgetrenntes Plasma trennt. Das Abflusssystem 15 umfasst eine Filtrationspumpe 16, einen Drucksensor 17 zur Messung des Filtrationsdrucks sowie einen Abfluss 18 zum Sammeln bzw. Entsorgen des abgetrennten Plasmas.

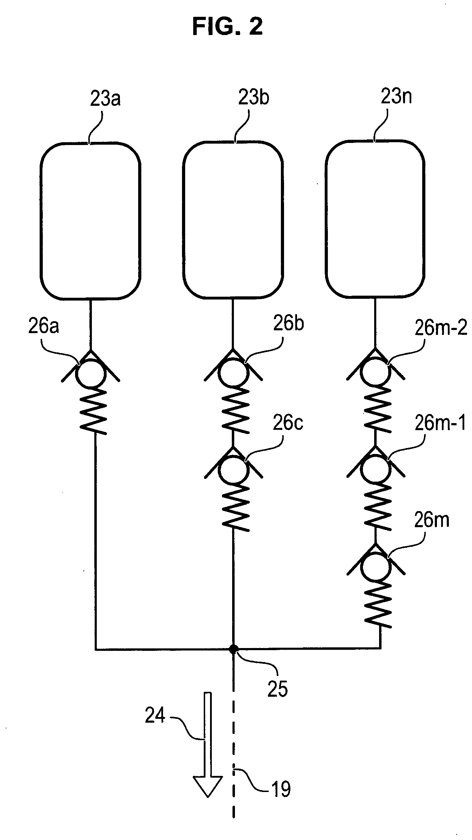

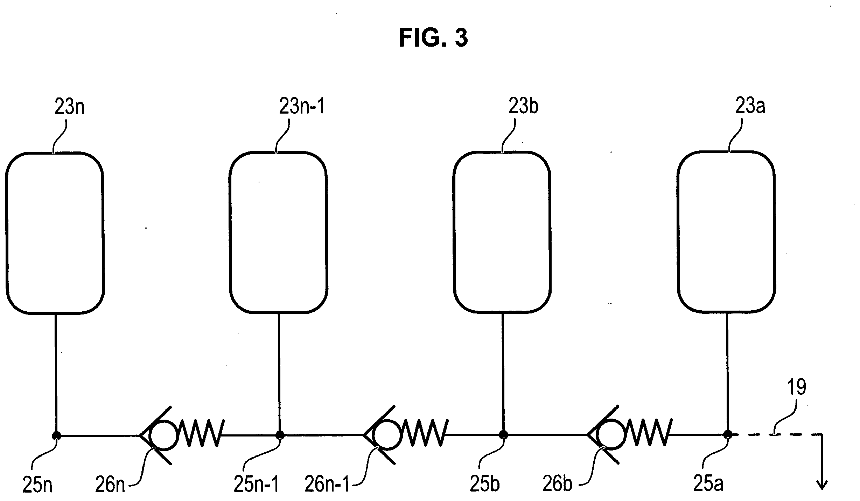

Ferner umfasst die Vorrichtung eine Substitutionsleitung 19, die stromaufwärts der Tropfkammer 12 oder an der Tropfkammer 12 in die venöse Leitung 4 des extrakorporalen Blutkreislaufs 1 mündet. Stromaufwärts der Substitutionsleitung 19 ist ein Versorgungssystem 20 für Substitutionsflüssigkeit angeordnet, auf das sich der Kerngedanke der vorliegenden Erfindung bezieht und das nachfolgend noch im Detail anhand der Figuren 2 und 3 diskutiert werden wird. In der Substitutionsleitung 19 ist ferner eine Substitutionspumpe 21 angeordnet, die der Förderung von Substitutionsflüssigkeit aus dem System 20 in die venöse Leitung 4 dient. Des Weiteren umfasst die Substitutionsleitung 19 noch Heizbeutel 22a und 22b, die einen Wärmetauscher umfassen und dazu dienen, die Temperatur der Substitutionsflüssigkeit auf Körpertemperatur anzuheben.

In Figur 2 ist eine erfindungsgemäß ausgebildete Ausführungsform eines Versorgungssystems 20 für Substitutionsflüssigkeit dargestellt. Das dargestellte Versorgungssystem 20 weist eine Mehrzahl von Kunststoffbeuteln 23a-n auf, die mit Substitutionsflüssigkeit wie beispielsweise Spenderplasma gefüllt sind. In der Figur sind drei Beutel gezeigt, das erklärte Prinzip ist aber auf Systeme mit beliebig vielen Beuteln anwendbar. Die Beutel 23a-n sind stromaufwärts der Pumpe 21 an die Substitutionsleitung 19 angeschlossen. Sie sind parallel geschaltet und münden an einem gemeinsamen Anschluss 25 in die Substitutionsleitung 19. Die Flussrichtung für Substitutionsflüssigkeit in der Substitutionsleitung 19 ist anhand des Pfeiles 24 gezeigt. Im Fluidkanal zwischen jedem der Beutel 23a-n und dem Anschluss 25 ist wenigstens ein Rückschlagventil 26 angeordnet. Diese Ventile 26a-m sind jeweils so ausgeführt, dass nach Erreichen eines bestimmten Öffnungsdrucks ein Fluss von Substitutionsflüssigkeit aus dem Beuteln 23 in die Substitutionsleitung 19 ermöglicht wird. Ein Fluss in die Gegenrichtung ist nicht möglich. In der Figur sind alle diese Ventile 26 identisch ausgeführt und öffnen sich insoweit auch alle bei Erreichen desselben Öffnungsdrucks. Sind mehrere Druckventile 26 in Serie zwischen einem bestimmten Kunststoffbeutel 23n und dem Anschluss 25 angeordnet, so addiert sich der Öffnungsdruck dieser seriell angeordneten Ventile zu einem Gesamtöffnungsdruck für einen bestimmten Beutel. Im gezeigten Beispiel wird der geringste Gesamtöffnungsdruck zur Entleerung des Beutels 23a benötigt, da zwischen diesem Beutel und dem Anschluss 25 nur ein Ventil 26a angeordnet ist.

Wird also die Substitutionsmittelpumpe 21 betrieben, erzeugt diese einen Unterdruck stromabwärts der Ventile 26, sodass sich das Ventil 26a bei Erreichen einer bestimmten Druckdifferenz öffnet und Substitutionsflüssigkeit aus dem Beutel 23a in die Substitutionsleitung 19 gelangen und in den extrakorporalen Blutkreislauf 1 gefördert werden kann. Aus den Beuteln 23b-n wird keine Substitutionslösung entnommen, solange der Beutel 23a nicht vollständig entleert ist. Ist der Beutel 23a vollständig entleert, erhöht sich bei einem kontinuierlichen Betrieb der Pumpe 21 automatisch der Unterdruck stromabwärts der Ventile 26, bis die Druckdifferenz dem Gesamtöffnungsdruck der Ventile 26b und 26c entspricht. Dann öffnen sich diese Ventile 26b und 26c und Substitutionsflüssigkeit kann aus dem Beutel 23b in die Substitutionsleitung 19 gelangen und in den extrakorporalen Blutkreislauf 1 gefördert werden. Dieses Prinzip wiederholt sich für die weiteren Beutel 23n und Ventile 26m entsprechend.

In einer Variante kann vorgesehen sein, in der Leitung zwischen einem der Beutel, beispielsweise dem Beutel 23a und dem Anschluss 25 gar kein Ventil anzuordnen. So kann aus diesem Beutel bereits ohne Differenzdruck Substitutionsflüssigkeit entnommen werden.

Ferner kann in einer alternativen Ausgestaltung vorgesehen sein, zwei oder mehrere unterschiedliche Ventile 26 einzusetzen und so die Gesamtzahl an benötigten Ventilen 26 zu verringern. Beispielsweise kann dann zwischen dem Beutel 23b und dem Anschluss 25 nur ein Ventil angeordnet sein, dessen Öffnungsdruck höher ist als der Öffnungsdruck des Ventils 23a.

Sollten zwei unterschiedliche Ventile verwendet werden, wobei ein erstes Ventil einen Öffnungsdruck von 50 mbar und ein zweites Ventil einen Öffnungsdruck von 100 mbar aufweist, ist beispielsweise die in Tabelle 1 gezeigte Konfiguration denkbar:

**Tabelle 1:**

| Beutelanschluss (n) | Ventil 1 | Ventil 2 | Gesamtöffnungsdruck [mbar] |
|---|---|---|---|
| 1 | 0 | 0 | 0 |
| 2 | 1 | 0 | 50 |
| 3 | 0 | 1 | 100 |
| 4 | 1 | 1 | 150 |
| 5 | 0 | 2 | 200 |
| usw. | | | |

In Figur 3 ist eine weitere Ausführungsform eines Versorgungssystems 20 gemäß der vorliegenden Erfindung dargestellt, wobei die Beutel 23a-n seriell an der Substitutionsleitung 19 angeschlossen sind, sodass die Anschlüsse 25a-n in Serie nacheinander an der Substitutionsleitung 19 liegen. Zwischen den Anschlüssen 25a-n sind jeweils Ventile 26b-n angeordnet, wobei es sich dabei vorzugsweise um Ventile handelt, die identisch ausgeführt sind und sich insoweit auch alle bei Erreichen desselben Öffnungsdrucks öffnen.

Wird in dieser Ausführungsform die Substitutionsmittelpumpe 21 betrieben, wird zunächst der erste Beutel 23a entleert, zwischen dem und der Pumpe sich kein Ventil 26 befindet. Ist dieser Beutel vollständig entleert, erzeugt die Pumpe 21 einen Unterdruck stromabwärts des ersten Ventils 26b, sodass sich dieses bei Erreichen einer bestimmten Druckdifferenz (beispielsweise 50 mbar) öffnet und Substitutionsflüssigkeit aus dem zweiten Beutel 23b in die Substitutionsleitung 19 gelangen und in den extrakorporalen Blutkreislauf 1 gefördert werden kann. Aus den Beuteln 23c-n wird weiterhin keine Substitutionslösung entnommen, da ein höherer Druckunterschied benötigt würde, um zusätzlich zum ersten Ventil 26b auch noch das zweite Ventil 26c zu öffnen. Ist nun auch der Beutel 23b vollständig entleert, erhöht sich bei einem kontinuierlichen Betrieb der Pumpe 21 der Unterdruck, bis die Druckdifferenz dem Gesamtöffnungsdruck der Ventile 26b und 26c entspricht. Dann öffnet sich auch das Ventil 26c und Substitutionsflüssigkeit kann aus dem Beutel 23c in die Substitutionsleitung 19 gelangen und in den extrakorporalen Blutkreislauf 1 gefördert werden. Dieses Prinzip wiederholt sich für die weiteren Beutel 23n und Ventile 26n entsprechend.

In der Ausführungsform gemäß Figur 3 ist vorteilhaft, dass die Anzahl der erforderlichen Ventile reduziert wird und gleichzeitig die Gefahr verringert wird, dass zwei Beutel gleichzeitig entleert werden, da die Öffnungsdrucke der Ventile nicht exakt aufeinander abgestimmt werden müssen.

## Patentansprüche

1. Vorrichtung zur Durchführung einer extrakorporalen Blutbehandlung, in der dem Patienten eine Substitutionsflüssigkeit verabreicht wird, wobei die Vorrichtung einen extrakorporalen Blutkreislauf (1) und eine in den extrakorporalen Blutkreislauf (1) mündende Substitutionsleitung (19) umfasst, wobei die Substitutionsleitung (19) eine Pumpe (21) zur Förderung von Substitutionsflüssigkeit in den extrakorporalen Blutkreislauf (1) aufweist und wobei wenigstens zwei und vorzugsweise mehr als zwei Behältnisse (23) zur Bereitstellung einer Substitutionsflüssigkeit separat und saugseitig der Pumpe (21) an die Substitutionsleitung (19) angeschlossen sind,
**dadurch gekennzeichnet,**
**dass** zwischen der Pumpe (21) und wenigstens einem der Behältnisse (23) wenigstens ein Druckventil (26) angeordnet ist, das bei Überschreitung eines Öffnungsdrucks einen Fluss von Substitutionsflüssigkeit zur Pumpe (21) erlaubt.

2. Vorrichtung nach Anspruch 1, **dadurch gekennzeichnet, dass** die Anordnung der Druckventile (26) und Behältnisse (23) derart ist, dass eine Abstaffelung der Öffnungsdrücke erreicht wird, welche von der Pumpe (21) ausgeübt werden müssen, um Substitutionsflüssigkeit aus unterschiedlichen Behältnissen (23) zu beziehen.

3. Vorrichtung nach einem der vorhergehenden Ansprüche, **dadurch gekennzeichnet, dass** die Druckventile (26) stromaufwärts der Anschlüsse (25) der jeweiligen Behältnisse (23) an die Substitutionsleitung (19) angeordnet sind.

4. Vorrichtung nach Anspruch 3, **dadurch gekennzeichnet, dass** jedem Behältnis (23) wenigstens ein gesondertes Druckventil (26) zugeordnet ist.

5. Vorrichtung nach Anspruch 3 oder 4, **dadurch gekennzeichnet, dass** unterschiedlichen Behältnissen (23) unterschiedlich viele in Serie geschaltete Druckventile (26) zugeordnet sind, die vorzugsweise alle denselben Öffnungsdruck aufweisen.

6. Vorrichtung nach Anspruch 3 oder 4, **dadurch gekennzeichnet, dass** unterschiedlichen Behältnissen (23) Druckventile (26) mit unterschiedlichen Öffnungsdrücken zugeordnet sind.

7. Vorrichtung nach einem der vorhergehenden Ansprüche, **dadurch gekennzeichnet, dass** einige oder alle der Behältnisse (23) parallel an die Substitutionsleitung (19) angeschlossen sind.

8. Vorrichtung nach einem Ansprüche 1 bis 6, **dadurch gekennzeichnet, dass** einige oder alle der Behältnisse (23) seriell an die Substitutionsleitung (19) angeschlossen sind.

9. Vorrichtung nach Anspruch 8, **dadurch gekennzeichnet, dass** die Druckventile zwischen den Anschlüssen (25) der Behältnisse (23) an die Substitutionsleitung (19) angeordnet sind.

10. Vorrichtung nach Anspruch 9, **dadurch gekennzeichnet, dass** zwischen der Pumpe (21) und dem ersten Behältnis (23a) der Serie kein Druckventil angeordnet ist und/oder dass alle Druckventile (23) denselben Öffnungsdruck aufweisen.

## Claims

1. An apparatus for carrying out an extracorporeal blood treatment in which a substitution fluid is administered to the patient, wherein the apparatus comprises an extracorporeal blood circuit (1) and a substitution line (19) opening into the extracorporeal blood circuit (1), wherein the substitution line (19) has a pump (21) for conveying substitution fluid into the extracorporeal blood circuit (1), and wherein at least two, and preferably more than two, containers (23) for providing a substitution fluid are connected separately and at the intake side of the pump (21) to the substitution line (19),
**characterized in that**
at least one pressure valve (26) is arranged between the pump (21) and at least one of the containers (23), said pressure valve allowing a flow of substitution fluid to the pump (21) on an exceeding of an opening pressure.

2. An apparatus in accordance with claim 1, **characterized in that** the arrangement of the pressure valves (26) and containers (23) is such that a staggering of the opening pressures is achieved which have to be exerted by the pump (21) to obtain substitution fluid from different containers (23).

3. An apparatus in accordance with one of the preceding claims, **characterized in that** the pressure valves (26) are arranged upstream of the connectors (25) of the respective containers (23) to the substitution line (19).

4. An apparatus in accordance with claim 3, **characterized in that** at least one separate pressure valve (26) is associated with each container (23).

5. An apparatus in accordance with claim 3 or claim 4, **characterized in that** a different number of pressure valves (26) connected in series, which preferably all have the same opening pressure, are associated with different containers (23).

6. An apparatus in accordance with claim 3 or claim 4, **characterized in that** pressure valves (26) having different opening pressures are associated with different containers (23).

7. An apparatus in accordance with one of the preceding claims, **characterized in that** some or all of the containers (23) are connected to the substitution line (19) in parallel.

8. An apparatus in accordance with one of the claims 1 to 6, **characterized in that** some or all of the containers (23) are connected to the substitution line (19) in series.

9. An apparatus in accordance with claim 8, **characterized in that** the pressure valves are arranged between the connectors (25) of the containers (23) to the substitution line (19).

10. An apparatus in accordance with claim 9, **characterized in that** no pressure valve is arranged between the pump (21) and the first container (23a) of the series; and/or **in that** all the pressure valves (23) have the same opening pressure.

## Revendications

1. Dispositif pour effectuer un traitement extracorporel du sang, dans lequel un liquide de substitution est administré au patient, le dispositif comprenant un circuit sanguin extracorporel (1) et un conduit de substitution (19) débouchant dans le circuit sanguin extracorporel (1), le conduit de substitution (19) comportant une pompe (21) pour le refoulement de liquide de substitution dans le circuit sanguin extracorporel (1) et au moins deux, et de préférence plus de deux contenants (23) pour la mise à disposition d'un liquide de substitution étant raccordés au conduit de substitution (19) séparément de la pompe (21) et côté aspiration de celle-ci,
**caractérisé en ce que**
au moins une soupape de refoulement (26) est disposée entre la pompe (21) et au moins un des contenants (23), ladite soupape de refoulement permettant un écoulement de liquide de substitution vers la pompe (21) lorsqu'une pression d'ouverture est dépassée.

2. Dispositif selon la revendication 1, **caractérisé en ce que** la disposition des soupapes de refoulement (26) et des contenants (23) est telle que l'on obtient un échelonnement des pressions d'ouverture qui doivent être exercées par la pompe (21) pour l'approvisionnement en liquide de substitution depuis différents contenants (23).

3. Dispositif selon l'une des revendications précédentes, **caractérisé en ce que** les soupapes de refoulement (26) sont disposées en amont des raccordements (25) des contenants (23) respectifs au conduit de substitution (19).

4. Dispositif selon la revendication 3, **caractérisé en ce qu'**au moins une soupape de refoulement (26) séparée est associée à chaque contenant (23).

5. Dispositif selon la revendication 3 ou 4, **caractérisé en ce qu'**un nombre différent de soupapes de refoulement (26) montées en série est associé à différents contenants (23), lesquelles présentent de préférence toutes la même pression d'ouverture.

6. Dispositif selon la revendication 3 ou 4, **caractérisé en ce que** des soupapes de refoulement (26) avec différentes pressions d'ouverture sont associées à différents contenants (23).

7. Dispositif selon l'une des revendications précédentes, **caractérisé en ce que** certains des contenants (23) ou tous sont raccordés en parallèle au conduit de substitution (19).

8. Dispositif selon l'une des revendications 1 à 6, **caractérisé en ce que** certains des contenants (23) ou tous sont raccordés en série au conduit de substitution (19).

9. Dispositif selon la revendication 8, **caractérisé en ce que** les soupapes de refoulement sont disposées entre les raccordements (25) des contenants (23) au conduit de substitution (19).

10. Dispositif selon la revendication 9, **caractérisé en ce qu'**aucune soupape de refoulement n'est disposée entre la pompe (21) et le premier contenant (23a) de la série et/ou **en ce que** toutes les soupapes de refoulement (23) présentent la même pression d'ouverture.
